(19) Europäisches Patentamt
European Patent Office
Office européen des brevets

(11) **EP 3 905 951 B1**

(12) **EUROPEAN PATENT SPECIFICATION**

(45) Date of publication and mention
of the grant of the patent:
**28.02.2024 Bulletin 2024/09**

(51) International Patent Classification (IPC):
**A61B 5/02** (2006.01)

(21) Application number: **20736018.1**

(22) Date of filing: **06.01.2020**

(52) Cooperative Patent Classification (CPC):
**A61B 5/4884; A61B 5/0044; A61B 5/02007;
A61B 5/02028; A61B 5/0261; A61B 5/1075;
A61B 5/1079; A61B 5/1118; A61B 5/222;
G16H 30/40; G16H 50/50;** A61B 2505/05;
A61B 2505/09; A61B 2576/023

(86) International application number:
**PCT/US2020/012437**

(87) International publication number:
**WO 2020/142791 (09.07.2020 Gazette 2020/28)**

(54) **VIRTUAL STRESS TEST BASED ON ELECTRONIC PATIENT DATA**

VIRTUELLER BELASTUNGSTEST BASIEREND AUF ELEKTRONISCHEN PATIENTENDATEN

TEST DE CONTRAINTE VIRTUEL BASÉ SUR LES DONNÉES DE PATIENT ÉLECTRONIQUES

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **06.01.2019 US 201962788911 P**

(43) Date of publication of application:
**10.11.2021 Bulletin 2021/45**

(73) Proprietor: **Covanos, Inc.
Atlanta GA 30350 (US)**

(72) Inventors:
• **GIDDENS, Don P.
South Carolina 29928-6927 (US)**
• **LEFIEUX, Adrien
57950 Montigny-les-Metz (FR)**
• **MOLONY, David
Atlanta, Georgia 30309 (US)**
• **VENEZIANI, Alessandro
Decatur, Georgia 30033 (US)**
• **SAMADY, Habib
Atlanta, Georgia 30319-1070 (US)**

(74) Representative: **Nordmeyer, Philipp Werner
df-mp Dörries Frank-Molnia & Pohlman
Patentanwälte Rechtsanwälte PartG mbB
Theatinerstraße 16
80333 München (DE)**

(56) References cited:
US-A1- 2014 058 715     US-A1- 2014 200 867
US-A1- 2015 324 962     US-A1- 2018 032 653
US-A1- 2018 089 829     US-A1- 2018 368 916
US-A1- 2018 368 916

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

**Description**

**CROSS-REFERENCE TO RELATED APPLICATION**

This application claims the benefit of and priority to United States Provisional Application No. 62/788,911, filed January 6, 2019.

**FIELD**

This disclosure is generally directed to determination and display of patient hemodynamic information based on noninvasive imaging and computational fluid dynamics, including determination and display of blood pressure within coronary arteries over a range of simulated patient activity levels.

**BACKGROUND**

Coronary heart disease (CHD) is the most common cause of death in the U.S., with estimated direct and indirect annual costs of hundreds of billions of dollars. CHD results from atherosclerosis, which can progress and lead to ischemia, angina, myocardial infarction and death. Various treatment options, including medical therapy, intravascular stents, and coronary artery bypass graft (CABG) surgery, can be provided to a patient depending upon the severity and complexity of the patient's lesions and clinical status. A typical diagnostic and treatment plan includes clinical evaluation, non-invasive stress testing and, for appropriate patients, invasive coronary angiography and subsequent medical therapy and/or coronary revascularization. Typically, if the patient remains symptomatic on medical therapy or a significant defect is found in myocardial perfusion, the care provider will perform an invasive coronary angiography on the patient. In such patients, the decision to revascularize or not using coronary stents or CABG surgery is made based on angiographic anatomical findings and, increasingly, with use of hemodynamic information, such as invasively-measured fractional flow reserve (FFR). Measurement of FFR in the catheterization laboratory requires inserting a pressure wire into the patient's coronary arteries, and an FFR value of less than 0.8 is generally considered to be indicative of a clinically-significant obstructive lesion warranting revascularization in the appropriate clinical context. Relevant prior art is disclosed in US2018/368916.

**SUMMARY**

Systems and methods for a virtual stress test based on electronic patient data are disclosed. An example embodiment of a computer-implemented method of performing a virtual stress test for a patient may include receiving medical image data of a patient, the medical image data including an arterial segment and generating a geometric representation of the arterial segment from the medical image data. The method may further include receiving metabolic requirement information of the patient and determining a threshold according to the metabolic requirement information. The method may further include determining a first flow field for the arterial segment using the geometric representation, the first flow field corresponding to a first activity level of the patient, determining a first pressure drop in the arterial segment based on the first flow field, determining a second flow field for the arterial segment using the geometric representation, the second flow field corresponding to a second activity level of the patient that is different from the first activity level, and determining a second pressure drop in the arterial segment based on the second flow field. The method may further include calculating, based on the first pressure drop and the second pressure drop, a range of pressure drops for a range of activity levels, comparing the range of pressure drops to the threshold, and displaying a result of the comparison.

In an embodiment, the first activity level is associated with a first flow rate and the second activity level is associated with a second flow rate that is different from the first flow rate, and calculating a range of pressure drops for the range of activity levels includes calculating a range of pressure drops for a range of flow rates.

In an embodiment, the range of flow rates is between the first flow rate and the second flow rate.

In an embodiment, calculating the range of pressure drops for the range of activity levels includes calculating the range of pressure drops according to a quadratic relationship between pressure drop and flow rate.

In an embodiment, displaying a result of the comparison includes displaying a plot of the range of pressure drops and the threshold.

In an embodiment, the first activity level is a resting state.

In an embodiment, the second activity level is a hyperemic state.

In an embodiment, the method further includes determining an activity level at which the pressure drop exceeds the threshold.

In an embodiment, the first and second flow fields comprise first and second flow velocity fields, respectively.

In an embodiment, determining the first flow field for the arterial segment using the geometric representation may include obtaining a first inflow rate respective of the arterial segment, calculating first outflow rates respective of the arterial segment according to the first inflow rate, and calculating the first flow field in the arterial segment according to the geometric representation, the first inflow rate, and the first outflow rates. In an embodiment, determining the second flow field for the arterial segment using the geometric representation includes obtaining a second inflow rate respective of the arterial segment, calculating second outflow rates respective of the arterial segment according to the second inflow rate, and calculating the second flow field in the arterial segment according to the geometric representation, the second inflow rate, and the second outflow rates.

An example embodiment of a computer-implemented method of performing a virtual stress test for a patient includes obtaining a geometric representation of an arterial segment of the patient, the arterial segment comprising an inflow boundary and two or more outflow boundaries, receiving metabolic requirement information of the patient, and determining a threshold according to the metabolic requirement information. The method may further include obtaining a first inflow rate respective of the inflow boundary, calculating first outflow rates at the two or more outflow boundaries according to the first inflow rate, calculating a first flow field in the arterial segment according to the geometric representation, the first inflow rate, and the first outflow rates, and determining a first pressure drop in the arterial segment based on the first flow field. The method may further include obtaining a second inflow rate respective of the inflow boundary, the second inflow rate different from the first inflow rate, calculating second outflow rates at the two or more outflow boundaries according to the second inflow rate, calculating a second flow field in the arterial segment according to the geometric representation, the second inflow rate, and the second outflow rates, and determining a second pressure drop in the arterial segment based on the second flow field. The method may further include calculating, based on the first pressure drop and the second pressure drop, a range of pressure drops for a range of inflow rates, comparing the range of pressure drops to the threshold, and displaying a result of the comparison.

In an embodiment, the first inflow rate is associated with a first activity level and the second inflow rate is associated with a second activity level that is different from the first activity level, and calculating a range of pressure drops for the range of flow rates includes calculating a range of pressure drops for a range of activity levels.

In an embodiment, the range of flow rates is between the first flow rate and the second flow rate.

In an embodiment, calculating the range of pressure drops includes calculating the range of pressure drops according to a quadratic relationship between pressure drop and inflow rate.

In an embodiment, displaying a result of the comparison includes displaying a plot of the range of pressure drops and the threshold.

In an embodiment, the method further includes determining an activity level at which the pressure drop exceeds the threshold.

In an embodiment, the first inflow rate is respective of a resting state of the patient.

In an embodiment, the second inflow rate is respective of a hyperemic state of the patient.

In an embodiment, the first and second flow fields comprise first and second flow velocity fields, respectively.

In an embodiment, the first and second flow fields are calculated according to three-dimensional, time-independent equations.

## BRIEF DESCRIPTION OF THE DRAWINGS

FIG. 1 is a diagrammatic view of an example embodiment of an electronic system for performing a virtual stress test.
FIG. 2 is a flow chart illustrating an example embodiment of a method for performing a virtual stress test based on electronic patient data.
FIG. 3 is a flow chart illustrating an example embodiment of a method for performing a virtual stress test based on electronic patient data.
FIG. 4 illustrates an example geometric models of a patient anatomical region that may be determined and find use with the methods of the present disclosure.
FIG. 5 is a plot illustrating example ranges of pressure drops for example ranges of activity levels for a set of patients.
FIG. 6 is a diagrammatic view of an example embodiment of a user computing environment.

## DETAILED DESCRIPTION

Screening for arterial lesions by non-invasive clinical diagnosis and traditional stress testing can be inaccurate, leading to unnecessary coronary angiography procedures for patients who have false positive tests, and can also result in false negatives in patients who have significant lesions. In a recent study, 55.3% of patients who had traditional noninvasive testing and went to invasive coronary angiography (ICA) had no obstructive CHD. In the U.S. alone, an estimated 1,115,000 inpatient cardiac catheterizations were performed in 2006, so a highly accurate noninvasive test that prevents unnecessary ICA procedures has the potential to save billions of dollars annually. Further, although ICA procedures are generally very safe for patients, negative outcomes do occur on rare occasions, and patient anxiety can be substantial, so reducing the number of unnecessary ICA procedures can improve outcomes and the patient experience. Further, studies demonstrate that deferring revascularization based on non-ischemic FFR values results in favorable outcomes relative to stenting, and FFR-based stenting results in approximately 30% reduction in the number of stents, death, heart attacks, and need for repeat stenting relative to standard angiographic guided care, as well as reducing costs.

Originally, FFR was defined as the ratio of maximum blood flow distal to a stenotic lesion to normal maximum flow in the same vessel. In clinical practice, however, FFR is usually defined as the pressure distal to a stenosis (Pd) relative to the aortic pressure (Pa) (*i.e.,* FFR = Pd/Pa) under hyperemic flow conditions. Because of the high percentage of unnecessary invasive angiograms, there has been great interest in developing a method to assess FFR noninvasively. From the underlying principles of physics, FFR is actually a variable that is dependent on the detailed hemodynamic flow field, which is a function of arterial geometry, pressures, and flow conditions. Thus, it is possible to calculate FFR from the fluid dynamic equations of motion, and this can be termed virtual FFR, or FFRv. This requires numerical procedures that are part of the field of computational fluid dynamics (CFD). CFD includes using detailed vessel geometry of the region of interest, as well as blood flow (or, alternatively, the imposed pressure) at the boundaries of the computational domain, to solve for the entire flow field and pressures within the region of interest (ROI).

Coronary artery flow requirements vary among individuals and depend upon several physiological factors, including age, gender, body mass index (BMI), and level of physical activity. To assess whether coronary flow is adequate in the patient, and hence that there are no significant coronary stenoses, exercise stress tests may be performed. This involves increasing levels of exercise which, in turn, results in increased heart rate, systolic blood pressure, and myocardial contractility. To sustain the exercise, coronary blood flow increases sufficiently to supply the myocardium to meet its increasing demand. If the patient has a significant epicardial stenosis, the autoregulatory reserve is outstripped and the corresponding myocardial bed becomes ischemic. This will manifest as symptoms of chest pain or dyspnea, electrocardiographic ST depressions, abnormal wall motion if adjunctive echocardiography is used, or abnormal myocardial perfusion if adjunctive myocardial perfusion imaging is performed. Although an indirect assessment of the presence of coronary stenosis, this approach provides a patient-specific estimate of the ischemic burden which informs the decision to perform further testing (*e.g.* angiography) and/or to revascularize the patient or not.

An alternative diagnostic approach is to perform a direct anatomic assessment of the coronary arteries using either invasive coronary angiography or non-invasive CT angiography. This provides information on the presence and extent of coronary artery disease, and is particularly helpful to exclude disease or diagnose severe obstruction. However, in the frequent scenario of intermediate coronary lesions (40-80% diameter stenosis), anatomic assessment cannot accurately assess whether a narrowing is flow limiting or ischemia provoking. To overcome this limitation of the anatomic assessment, invasive FFR and noninvasive virtual FFR have been developed to compliment the anatomic information. Invasive FFR has become the standard for functional assessment of coronary lesions and does incorporate some patient-specific physiologic data. For instance, the response of a myocardial bed to hyperemic agents administered during invasive FFR measurement reflects the individual's coronary microvascular function. In contrast, for virtual FFR an assumed flow rate is typically prescribed to a proximal vessel supplying a myocardial bed. However, the true blood flow rate for a given stenosis is dependent on the patient's microvascular function and cardiopulmonary capacity (*e.g.*, an active 30 year old patient will have a much higher flow rate than an 85 year old wheelchair-bound patient, even if the two patients have similar anatomy). Accordingly, current computational methods for assessing patient coronary stenosis do not fully account for patient-specific coronary flow rates to inform calculations and decisions.

In order to provide a more patient-specific computational hemodynamic assessment, one or more blood flow physiological indices that describe pressure losses, such as FFR or instantaneous wave-free ratio (IWFR), may be calculated under a range of simulated activity levels or metabolic demands. Estimated or calculated information regarding the patient's physical and metabolic activity level can be used to inform a patient-specific computed FFR or IWFR for a given coronary lesion. The patient's specific physical activity or metabolic data can be obtained, for example, from a validated activity or lifestyle questionnaire. For example, such a questionnaire may enquire what activities the patient engages in, and the metabolic demand associated with those activities may be determined. An example methodology for associating a metabolic demand with an activity is described in Sidney et al., Metabolic Equivalents (METS) in Exercise Testing, Exercise Prescription, and Evaluation of Functional Capacity, 13 Clin. Cardiol. 555-565 (1990). Briefly, the metabolic demand associated with a given activity may be defined as a multiple of a metabolic equivalent (MET), which is the amount of oxygen a patient consumes at rest. The metabolic demand may range from one (1) MET (at rest) to over fifteen (15) METS for certain intense aerobic activities. The value of a single MET may be patient-specific and may depend on the body weight of the patient. The number of METS for a given activity may depend on the intensity and difficulty of the particular activity. A single MET may be defined as a volume of oxygen per minute, in an embodiment. Because the volume of oxygen per minute may scale linearly with the patient's flow rate, a direct relationship may be established between a patient's blood flow rate at a given point in the vasculature and the metabolic demand (*i.e.*, number of METS) served by that flow.

In addition to or as an alternative to determining metabolic demand based on an activity questionnaire, the patient's metabolic demand may be determined from standardized exercise tests such as Bruce protocols that many patients with suspected coronary heart disease undergo as part of their pre-procedure diagnostics. An example protocol for such exercise testing is described in Lear et al., Exercise Stress Testing: An Overview of Current Guidelines, 27(5) Sports Med. 285-312 (May 1999). Briefly, based on an exercise test, a patient's specific maximum MET value can be determined, *i.e.,* the MET value associated with the maximum level of activity that the patient participates in.

This patient specific metabolic work load may be used to inform the coronary flow rates (e.g., one or more boundary conditions) prescribed in the hemodynamic calculations and improve the diagnostic accuracy of the computed FFR value or other hemodynamic value(s) relative to known methods.

The Navier-Stokes equations can be employed to describe the flow field in a coronary artery, e.g., intravascular pressures and velocities in a region of interest (ROI) as functions of time and three-dimensional (3D) space, in some forms, and solely of 3D space in other forms. From these flow fields, quantities of clinical interest can be computed such as, for example, pressure drop, FFR, instantaneous wave-free ratio (IWFR), forces on artery walls caused by intravascular pressure variations and viscous shearing stresses (wall shear stress, WSS), etc. In order to solve the Navier-Stokes equations, CFD is employed, and the solution includes imposing boundary conditions for the ROI. The subject's vessel lumen geometry (obtained from CT or other vascular imaging) is also included in CFD, along with some combination of inflow rate (*e.g.,* at a selected inflow boundary) and flow distribution among vessel branches (*e.g.,* one or more outflows). The pressure field in the ROI may be computed as a deviation from a reference pressure, and thus the absolute level of pressure, *e.g.,* Pa, in the ROI may not be needed for CFD at the time of calculation of the pressure field. Once the deviations from reference are computed, the absolute pressures in the field can be determined when the reference pressure (*e.g.,* Pa) is determined. It should be noted, however, that not all pressure-related determinations-such as some embodiments of a virtual stress test disclosed herein-require a reference pressure, and can instead be made based on the pressure field without a baseline reference.

For many clinical applications in coronary artery flows, such as pressure drop, FFR and IWFR, the Navier-Stokes equations may be treated as independent of time (*i.e.,* in three spatial dimensions, but independent of the time dimension). For example, the time average of the pressure ratio, Pd/Pa, is representative of the average of the instantaneous pressure ratio, where Pd is the pressure in the ROI and Pa is the reference pressure. This means that a three-dimensional CFD model is appropriate for computing these pressure indices, thus enabling a faster computation than a four-dimensional model (*i.e.,* a model that incorporates three spatial dimensions and time).

A pressure field within a ROI may be determined based on, e.g., flow rates in and around the ROI. For coronary artery flow and pressure, the relationship between flow rate and pressure gradient ($\Delta P$) between a proximal location and a distal location in the region of interest can be approximated well by a quadratic equation, shown as equation (1) below:

$$\Delta P = aQ + bQ^2 \qquad\qquad (Eq.\ 1)$$

where a and b are constants that depend upon the vessel geometry and blood viscosity of an individual patient, and which may be calculated for a given patient in the manner described below. Equation (1) has both a physical and mathematical basis. Physically, the aQ term is related to pressure losses directly due to blood viscosity while the $bQ^2$ term is related to pressure losses arising from flow separation and, if present, turbulence. The $bQ^2$ term may be significant when a stenosis is sufficiently great to cause flow separation. Mathematically, the equation can be viewed as the first two terms in a polynomial series expansion for $\Delta P = fcn(Q)$. Equation (1) solves for the pressure drop across a region of a subject patient's vasculature for which a flow field is determined via computational fluid dynamics.

In order to compute a and b, the three-dimensional Navier-Stokes equations may be solved for two different values of Q. For example, a first value, $Q_1$, may represent a flow rate typical of resting conditions and a second value, $Q_2$, may represent a flow rate typical of an exercise (or hyperemic) state. These computations will give two values for $\Delta P$ so that equations (2) and (3), below, may be solved for a and b once $Q_1$, $Q_2$, $\Delta P_1$ and $\Delta P_2$ are known:

$$\Delta P_1 = aQ_1 + bQ_1{}^2 \qquad\qquad (Eq.\ 2)$$

$$\Delta P_2 = aQ_2 + bQ_2{}^2 \qquad\qquad (Eq.\ 3)$$

Once coefficients a and b are known for a given patient, it is possible to compute $\Delta P$ over a range of flow conditions (*e.g.,* a range of physiologically relevant flow conditions) without further CFD for each flow condition in the range.

In addition to geometric and flow split uncertainties, there are two sources of uncertainty in noninvasively computing FFR: (1) Pa, the intravascular aortic pressure under hyperemia, is not known; and (2) Q, the inflow under hyperemia, is not known.

CFD provides $\Delta P$ directly, independently of Pa. On the other hand, FFR (computed as the ratio Pd/Pa) depends directly on the patient-specific value of Pa. As a result, for computed FFR, a value of Pa must be assumed once $\Delta P$ is computed. The assumed value of Pa may be derived from a cuff blood pressure measurement, for example, or may be assumed to be Pa = 100 mmHg across all subjects.

There are clinical data to suggest that $\Delta P$ can be used as a diagnostic index for obstructive CAD. To demonstrate this plausibility, consider a population average of Pa = 100 mmHg. For such an average Pa value: (a) FFR = 0.8 (generally considered to indicate an obstructive lesion) corresponds to $\Delta P$ = 20 mmHg; and (b) IWFR = 0.9 (also generally considered to indicate an obstructive lesion) corresponds to $\Delta P$ = 10 mmHg.

There is thus a rationale for employing computational methods as a virtual stress test in the following scheme: (1) based on images of the patient ROI, such as CT images or other noninvasively-obtained images, compute $\Delta P$ at two different assumed flow rates; (2) employ the quadratic relationship set forth in equations (1) and (2) above to determine coefficients a and b for a subject; (3) present a curve of $\Delta P$ vs Q over a range of flow rates; (4) determine the flow rate that corresponds to a critical value of $\Delta P$ (e.g., 20 mmHg for FFR = 0.8; 10 mmHG for IWFR = 0.9); and (5) based on the patient's expected flow requirements at rest and under anticipated lifestyle activity, determine if the resulting $\Delta P$ values are indicative of obstructive CAD. This methodology will be discussed in greater detail below.

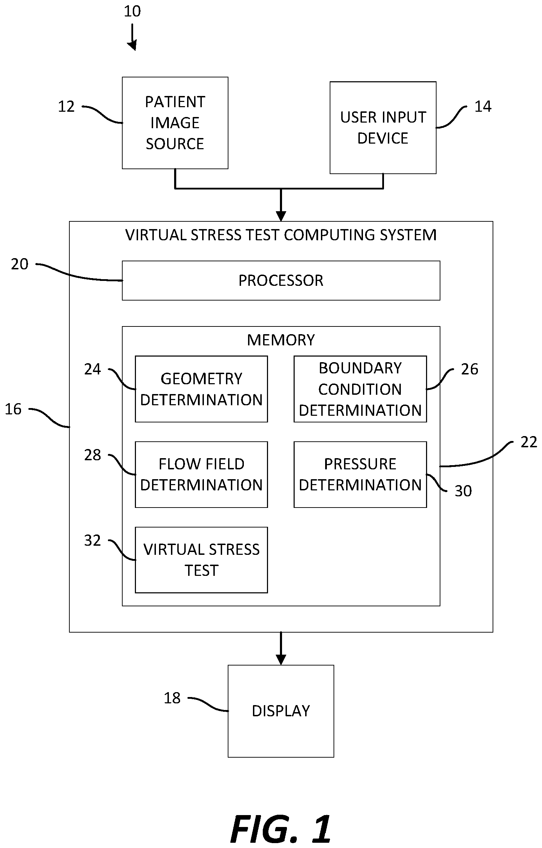

Referring to the drawings, wherein like reference numerals refer to the same or similar features in the various views, FIG. 1 is a diagrammatic view of an example embodiment of an electronic system 10 for performing a virtual stress test. The example system 10 may include a patient image source 12, a user input device 14, a virtual stress test computing system 16, and a display 18. As will be described in greater detail below, the system 10 may find use in performing a virtual stress test based on electronic patient data (e.g., images of a region of interest of the patient and other data) to determine various hemodynamic information about the patient, and/or to make a recommendation regarding further testing, interventional evaluation, and/or interventional therapy for the patient.

One or more aspects of the system 10 may be deployed in a clinical environment, in an embodiment. For example, in some embodiments, the patient image source 12, user input device 14, virtual stress test computing system 16, and the display 18 may all be provided in a common clinical setting, such as a hospital. In some embodiments, the components of the system 10 may be embodied in a laptop or desktop computer or workstation. In an embodiment, one or more components of the system, such as the virtual stress test computing system 16, may be provided remotely from the clinical setting, such as in a cloud computing service deployment.

The patient image source 12 may include a medical image acquisition device configured to acquire one or more medical images of a vascular system of a subject patient. For example, the patient image source 12 may be a noninvasive image acquisition device. In some embodiments, the patient image source 12 may include but is not limited to a computed tomography (CT) acquisition device, intravascular ultrasound (IVUS), biplane angiography, optical coherence tomography (OCT), magnetic resonance imaging (MRI), among others, or a combination thereof.

Additionally or alternatively, the patient image source 12 may include a store of existing image data. In some embodiments, patient image source 12 may include a medical image storage device, such as a database or other local electronic data storage, or a remote storage (e.g., cloud-based storage) configured to store medical images.

The user input device 14 may be or may include one or more devices for input to a computing system, such as a mouse, touchpad, touchscreen, keyboard, microphone, camera, or other input device.

The virtual stress test computing system 16 may include a processor 20 and a non-transitory, computer-readable memory 22 configured to store data and instructions. In an embodiment, the memory 22 may store images from a subject patient, and thus may serve as the patient image source 12, or an aspect thereof. The processor 20 may be configured to execute instructions stored in the memory 22 to perform one or more of the steps, methods, algorithms, etc. of this disclosure. In particular, the memory 22 may be configured to store various functional modules in the form of instructions, including a geometry determination module 24, a boundary condition determination module 26, a flow field determination module 28, a pressure determination module, and a virtual stress test module 32.

The various modules 24, 26, 28, 30, 32 in the memory 22 will be described separately, but it should be understood that such separation is for ease of discussion only. The instructions in which the various modules are embodied may be in common files, storage devices, etc. and, similarly, one or more of the modules described herein may be separated into multiple separate files, storage devices, etc.

The geometry determination module 24 may be configured to generate an electronic geometrical representation (*e.g.*, model) of an anatomical region of interest (ROI) from images obtained from the patient image source. In some embodiments, the ROI may be a prtion of the subject patient's cardiovascular system, such as one or more arterial segments. The one or more arterial segments may include a portion of one or more arteries and one or more branches that extend therefrom.

In some embodiments, the one or more arterial segments may include one or more coronary arterial segments. The one or more coronary arterial segments may include a portion of one or more coronary arteries emanating from an aorta of a subject and one or more branches that extend therefrom. The one or more coronary arterial segments may include but is not limited to one or more portions of the left coronary artery (LCA) and/or the right coronary artery (RCA). The one or more coronary arterial segments for the left coronary artery (LCA) may include but is not limited the left main coronary artery (LM), the left anterior descending (LAD), the left circumflex artery (also referred to as the "Circumflex"), among others, or a combination thereof.

The disclosure will make reference to coronary arterial segments. However, it will be understood that the one or more arterial segments are not limited to the coronary arterial segments discussed and may include other coronary arterial segments, other types of arterial segments, among others, or a combination thereof. For example, the one or more arterial segments may include cerebral arterial segment(s), femoral arterial segment(s), iliac arterial segment(s), popliteal arterial segment(s), carotid arterial segment(s), renal artery segments, and the like.

In some embodiments, the geometrical representation produced by the geometry determination module 24 may be a three-dimensional (3-D) electronic model of the spatial volume of one or more arterial segments. For example, the geometrical representation of one or more arterial segments may be discretized into a three-dimensional volumetric mesh, for example, polyhedrons (e.g., tetrahedrons). In some embodiments, the geometrical representation may include a surface mesh representing the boundary of the lumens of each arterial segment.

In some embodiments, the boundary condition determination module 26 may be configured to determine boundaries for each arterial segment. "Boundaries" may refer to cross-sections of the representation of the arterial segment and may include but are not limited to: inflow boundary corresponding to the cross-section through which the blood flows; one or more outflow boundaries corresponding to the cross-section disposed downstream or distal from the inflow boundary through which blood flow is directed outward; one or more vessel wall boundaries corresponding to an interface between the inner surface of the arterial wall and the flowing blood; among others; or combination thereof.

In some embodiments, the one or more outflow boundaries may include an outflow boundary disposed at or adjacent to a junction point (e.g., bifurcation, trifurcation, and the like, and combinations thereof). In some embodiments, the one or more outflow boundaries may include an outflow boundary disposed at or adjacent to the left Circumflex artery. In some embodiments, the one or more outflow boundaries may include a first outflow boundary and a second outflow boundary that is disposed between the inflow boundary and the first outflow boundary. In some embodiments, the first outflow boundary may correspond to a distal boundary of the segment (i.e., the cross-section disposed downstream or distal from the inflow boundary). In some embodiments, for example, when the geometrical representation includes the left coronary artery, the second outflow boundary may correspond to the circumflex. In some embodiments, the first outflow boundary and the second outflow boundary may be separated by one or more additional outflow boundaries, for example, at least a third outflow boundary. The third outflow boundary may correspond to or be adjacent to a junction point, such as a branch or bifurcation.

In some embodiments, the boundary condition determination module 26 may be configured to determine geometrical data for each boundary using the geometric representation generated by the geometry determination module 24. In some embodiments, the geometrical data may include but is not limited to vessel radius, diameter, circumference, area, epicardial coronary volume, length, among others, or a combination thereof.

In some embodiments, the boundary condition determination module 26 may be configured to determine boundary conditions for each boundary for each arterial segment. By way of example, the boundary conditions for each segment may include inflow boundary conditions, outflow boundary conditions, one or more vessel wall boundary conditions, among others, or a combination thereof. The inflow boundary condition may be a value or a range of values for velocity, flow rate, pressure or other characteristics. Each outflow boundary condition may be a value or a range of values for velocity, flow rate, pressure, a percentage of inflow boundary, or other characteristic. Each vessel wall boundary condition may be a value or a range of values for velocity, flow rate, pressure, a combination thereof, or other characteristic.

In some embodiments, the determination of the inflow boundary condition and/or outflow boundary conditions may be determined based on patient information, an applicable physiological state (*e.g.*, resting state, hyperemic state), the type of segment (e.g., LCA or RCA), among others, or a combination thereof. In some embodiments, an inflow boundary condition may be determined according to an expected patient activity level (e.g., based on information provided by the patient). In some embodiments, the inflow boundary condition may be a stored value and/or specified by the user.

In some embodiments, an inflow boundary condition may be determined according to the geometry of the anatomical ROI, such as the radius, diameter, length, or volume of a vessel portion. For example, the flow rate may be calculated according to a model based on the lumen volume of the region of interest, as shown in equation 4 below:

$$Q_{in} = \alpha V^{\beta} \qquad\qquad (Eq.\ 4)$$

where $Q_{in}$ is a flow rate at an inlet of the anatomical model, V is the lumen volume of the region of interest, $\alpha$ is a

coefficient that depends on the physiologic state of the patient, and β is a coefficient that depends on the vessel tree structure and, in some embodiments, resolution of the images used to generate the 3D model of the anatomical region.

In embodiments in which the region of interest is the coronary artery tree, V may be the lumen volume of the LCA or RCA defined from the proximal origin to a location where the segmented vessel diameter is a particular diameter, which diameter may depend on the resolution of the images used to create the model of the patient anatomical portion. For example, the location may be defined as the diameter of three or four voxels in the image data set, in some embodiments. In a particular example, the location may be where the lumen has a diameter of 1 mm or 1.5 mm.

In some embodiments, parameters α and β may be constants across all patients and may be determined from an example data set having both noninvasive and invasive data from which the values of α and β may be validated.

In some embodiments, the outflow boundary conditions may be determined using an outflow distribution model. The outflow distribution model may be determined using geometrical data and/or stored hemodynamic data. The stored hemodynamic data may define or be used to define an empirical relationship between geometry (*e.g.*, radii, diameters, lengths, volumes, or other geometric characteristics) of outflow boundaries and respective flow rates. For example, the boundary condition generation module can determine the outflow distribution model using stored hemodynamic data and the radii, diameters, lengths, volumes, and/or other geometric features of the first and second outflow boundaries of the segment. In another example, the boundary condition generation module can determine the outflow distribution model using only geometrical data, for example, the radius, diameter, length, volume, and/or other geometric features of the first outflow boundary (the distal boundary) of the segment. The outflow distribution model can be used to determine outflow (e.g., velocity, flow rate, percentage of inflow) for each outflow boundary, thereby determining each outflow boundary condition.

By way of example, the boundary conditions determined by the boundary condition determination module 26 can be used with steady and/or unsteady flow computations to determine flow field (e.g., blood flow, wall shear stress, etc.) and hemodynamic information (e.g., FFR, IWFR, etc.). The boundary condition determination module also uses an optimization approach to define the artery segment flow splitting. Therefore, the boundary condition generation module 26 can provide flexibility, accuracy, and efficiency in determining the boundary conditions.

The flow field determination module 28 may be configured to determine a flow field for each arterial segment using the geometrical representation determined by the geometry determination module 24, the one or more boundary conditions determined by the boundary condition determination module 26, and pressure data respective of the patient. The pressure data may be, for example, a cuff pressure of the patient at a state of rest. In some embodiments, the flow field may include but is not limited to pressure field, velocity field, wall shear stress field, axial plaque stress, among others, or a combination thereof.

In some embodiments, a flow field parameter (e.g., pressure field, velocity, etc.) may be based on only the geometrical data and boundary conditions. This way, the flow field determination module may be configured to determine the flow field based only spatial location (i.e., independent of time).

The pressure determination module 30 may be configured to determine blood pressure at one or more points in a patient anatomy using the flow field determined by the flow field determination module 28. In some embodiments, the pressure data can be determined from a computed flow/pressure field, a non-invasive determination of a mean blood pressure of the patient, for example, determined by a blood pressure cuff, among others, or a combination thereof.

The pressure determination module 30 may be configured to determine a specific pressure at a specific location in the patient's anatomy responsive to a user (*e.g.*, physician) selection of the specific location. The user may enter that selection with the user input device 14 relative to a display of the geometric model of the patient region of interest (*e.g.*, arteries) on the display 18. In embodiments, the pressure determination module may be configured to determine pressures upstream and downstream from the user-selected location, so as to determine a pressure drop at the user-selected location.

The virtual stress test module 32 may be configured to simulate a stress test, *i.e.,* perform a virtual stress test, on the patient anatomy. The virtual stress test may include, in embodiments, calculating a range of pressure drops for one or more points in a patient's anatomy for a range of activity levels as disclosed herein. Based on the results of the virtual stress test, the virtual stress test computing system may determine an activity level for the patient at which the pressure drop indicates a clinically-significant stenosis in the patient's anatomy. Based on the determined level of activity, it can be determined by the system 16 or by a clinician whether further diagnostic procedures and/or interventional procedures should be performed.

In some embodiments, a virtual stress test performed by the virtual stress test module 32 may be performed on the basis of a pressure gradient within the anatomical region of interest, without determination of an absolute pressure, a reference pressure, or an absolute pressure-based hemodynamic index. In other embodiments, a virtual stress test may include determination of a reference pressure and calculation of one or more hemodynamic index values, such as IWFR, FFR, dPR, etc.

To calculate an absolute pressure or an absolute pressure-based hemodynamic index, one or more Pa values, such as a mean diastolic aortic pressure, may be calculated. In an embodiment, brachial cuff pressure measurements can be used to estimate the mean diastolic pressure. Cuff pressures provide peak systolic pressure (SP) and minimum diastolic pressure (DP). The resting mean aortic diastolic pressure ($Pa_{dmean}$, or dPa)-which may be used for an IWFR calculation-during the wave free period may be estimated from cuff values of SP and DP according to the transfer function set forth in the following equation 5, in some embodiments:

$$Pa_{dmean} = (SP + 3DP)/4 \qquad\qquad (Eq. 5)$$

In other embodiments, other transfer functions may be used to relate a cuff pressure values to a reference pressure.

In another embodiment, the resting mean aortic diastolic pressure Pa ($Pa_{dmean}$, or dPa) may be calculated from a cuff pressure as shown in equation 6 below:

$$dPa = Pc + offset \qquad\qquad (Eq. 6)$$

where Pc is the resting brachial cuff pressure given by equation 7 below:

$$Pc = dPc + FF*PP \qquad\qquad (Eq. 7)$$

where dPc is the diastolic cuff pressure, FF is a scalar form factor, and PP is the cuff pulse pressure (*e.g.*, the difference between the systolic (SP) and diastolic (DP) cuff pressures of the subject patient at rest). The scalar form factor FF may have a value of between 0.15 and 0.45, and may depend patient-specific characteristics, including heart rate, age, height, systolic pressure, and/or augmentation index, for example. In some embodiments, FF may be approximately 0.2, 0.25, or 0.33. In some embodiments, the offset value of equation 6 may be between about zero (0) and -10 mmHg. In an embodiment, the offset value of equation 6 may be about -7 mmHg. The value of the offset may depend on the value of the form factor FF, the desired hemodynamic index (and, therefore, the portion of the cardiac cycle under examination), and the physiological state of the patient, in some embodiments.

In other embodiments, the mean aortic diastolic pressure may be estimated using a transfer function that relates cuff pressures to aortic pressures during diastole. For example, the diastolic Pa value may be determined from a cuff pressure of the subject patient by finding a value of form factor FF and/or an offset value that fits a data set including invasively measured cuff pressures and diastolic resting central pressures of a patient population. Once this function is known, it can be used to obtain an estimate of the diastolic resting pressures from non-invasively measured cuff pressures.

In another embodiment, the mean aortic diastolic pressure may be estimated from a combination of an optical finger device or other wearable pressure measurement device (e.g., a radial tonometry device) and a brachial cuff pressure device. For example, a Fourier analysis of the optical finger device output may be performed and mathematically combined with the brachial cuff pressure to determine a value of Pa.

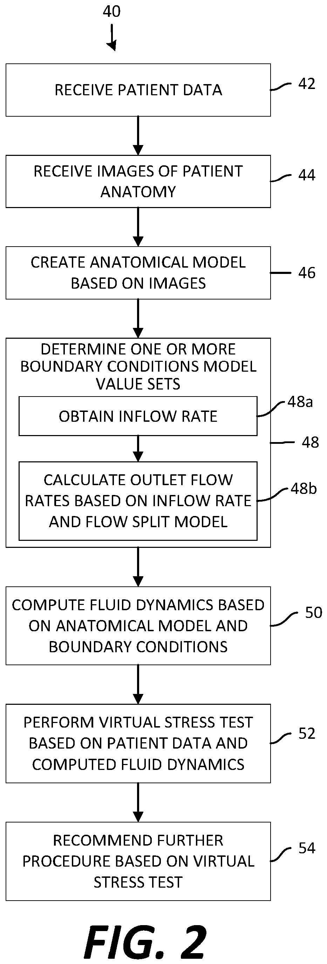

FIG. 2 is a flow chart illustrating an example embodiment of a method 40 for performing a virtual stress test. The method 40, or one or more aspects of the method 40, may be performed by the virtual stress test computing system 16 of FIG. 1, in embodiments.

The method 40 may include, at block 42, receiving patient data. The patient data may include, for example, basic information about the patient, such as the patient's age, gender, brachial cuff blood pressure, a description of user symptoms, etc. The patient data may further include, in embodiments, metabolic data of the patient, such as a user's typical activity level (e.g., sedentary or active, amount of exercise per week, amount of specific activities per week, such as walking and running, etc.). The patient data may further include, in an embodiment, patient data from one or more diagnostic tests, such as echocardiography.

The method 40 may further include, at block 44, receiving images of the patient anatomy. The received patient images may be CT images, MRI images, or other noninvasively-obtained images, in embodiments. The images may include an anatomical region of interest of the patient. In an embodiment, for example, the received images may include one or more coronary arteries or other vasculature of interest. The images may be received from a patient image source, such as an imaging device or a database or other computer memory.

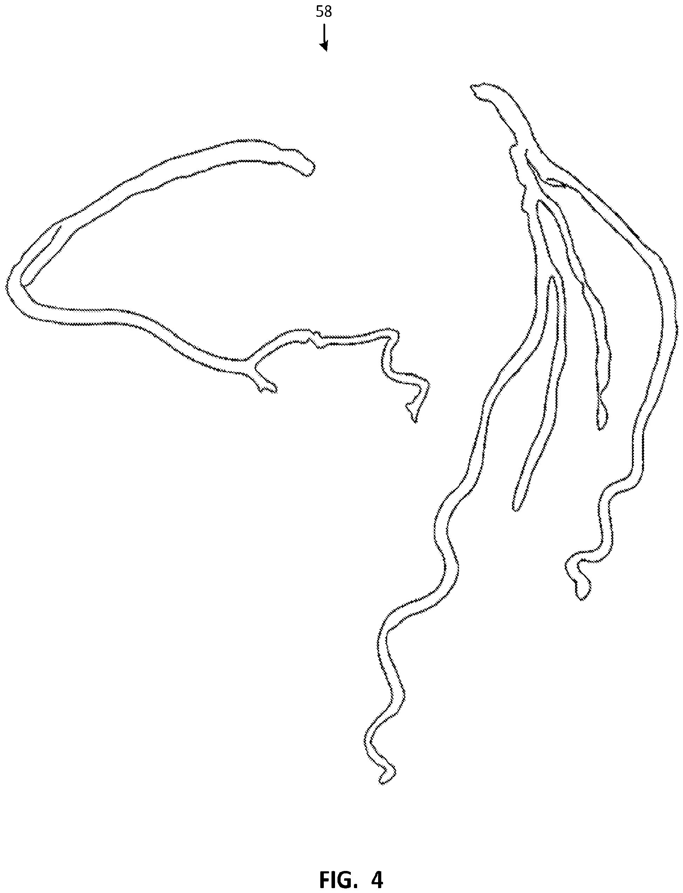

The method 40 may further include, at block 46, creating an anatomical model respective of the patient region of interest based on the images received at block 44. In an embodiment, the anatomical model may be created by segmenting the anatomy of interest from the images received at block 44. The anatomy of interest may be, for example, one or more coronary arteries. FIG. 4 illustrates an example anatomical model 56 of coronary arteries. The anatomical model may be created by the geometry determination module 24 of FIG. 1, in an embodiment. In some embodiments, an anatomical model may be obtained by a computing system (*e.g.*, the hemodynamic information computing system 16) by being created by the computing system, or by receiving an existing model of the subject patient.

The method 40 may further include, at block 48, determining one or more boundary conditions model value sets. The one or more boundary conditions value sets may be respective sets of values for the same boundary conditions model, in an embodiment (*e.g.*, respective sets of condition values for the same inflow boundaries, outflow boundaries, wall boundaries, etc.). Block 48 may include sub-parts 48a and 48b, in some embodiments. Accordingly, the method 40 may include, at block 48a, obtaining an inflow rate. The inflow rate may be a flow rate for an inlet of the anatomical ROI of the patient, in some embodiments. In other embodiments, the blood flow rate may be a flow rate for another portion of the ROI.

The inflow rate obtained at block 48a may be representative of a particular activity level or physiologic state of the patient. For example, in some embodiments, the inflow rate obtained at block 48a may be representative of a resting state of the patient. In another embodiment, the inflow rate may be representative of a hyperemic state of the patient. In still other embodiments, the inflow rate obtained at block 48a may be representative of an arbitrary activity level of the patient (*e.g.*, any feasible flow rate for the patient given the patient's age, health, etc.).

The inflow rate obtained at block 48a may be representative of a desired point or portion of the cardiac cycle of the patient, in some embodiments. For example, in some embodiments, the inflow rate obtained at block 48a may be representative of an average flow rate over the entire cardiac cycle of the patient, or over the entire wave-free period of diastole of the patient, or over the entirety of diastole of the patient. In other embodiments, the inflow rate may be representative of the particular time point within the cardiac cycle, such as the midpoint of diastole.

In some embodiments, obtaining the inflow rate at block 48a may include calculating an inflow rate according to a geometry of the patient anatomical model as discussed with respect to equation (4) above. As discussed above with respect to equation (4), the inflow rate $Q_{in}$ includes an $\alpha$ term which depends on the physiological state of the patient. Accordingly, obtaining an inflow rate at block 48a may include determining or selecting a value of $\alpha$ that is appropriate for the desired activity level.

In other embodiments, instead of calculating an inflow rate according to equation 4 above, obtaining a blood flow rate at block 48a may include receiving a user input of an inflow rate. The inflow rate may be received via user manual entry (*e.g.*, with the user input device 14 of the system 10). In an embodiment, the inflow rate at block 48a may be determined (*e.g.*, by a clinician or by an electronic system) based on the metabolic demands and condition of the patient.

Block 48 may further include, at block 48b, calculating outlet flow rates according to the inflow rate obtained at block 48a and according to a flow splitting model (which may also be referred to herein as an outflow distribution model). The flow splitting model may be, or may have been, calculated or otherwise determined according to a geometry of the three-dimensional electronic model of the patient anatomical region. The flow splitting model may be calculated according to the relative radii, diameters, circumferences, lengths, volumes, and/or surface areas of the vessels in the electronic model, in some embodiments.

In conjunction with the flow rate obtained in block 48a, the outlet flow rates calculated at block 48b may be comprise a boundary condition model value set respective of the patient anatomical region. The boundary condition model value set may be representative of a particular physiologic state or activity level of the patient (*e.g.*, a resting state, a hyperemic state, or other state) and a particular portion or point in the cardiac cycle of the patient (*e.g.*, the entire cardiac cycle, diastole, a time point in the cardiac cycle, etc.).

In embodiments in which the anatomical region is a coronary artery of the patient, the inflow rate obtained at block 48a may be an inlet flow rate for the coronary artery, and the flow splitting model may be calculated according to the geometry of the coronary artery portions downstream of the inlet in the electronic model. The flow splitting model may be calculated according to the relative radii, diameters, circumferences, lengths, surface areas, or volumes of the coronary artery portions downstream of the inlet. In some embodiments, the flow splitting model may be calculated according to the epicardial volume of the electronic model.

The method 40 may further include, at block 50, computing fluid dynamics of the blood flow through the patient anatomy based on the anatomical model (*e.g.*, model 56), the one or more boundary conditions model value sets, and, in some embodiments, the patient data. Block 50 may include determining the flow field for each arterial segment using the anatomical model, boundary conditions, and pressure data respective of the patient (e.g., aortic pressure data). In some embodiments, the pressure data may be obtained for the patient, for example, cuff pressure, and/or may be a stored value. In some embodiments, the flow field may include but is not limited to a pressure field, velocity field, among others, or a combination thereof. The fluid dynamics may be computed by the flow field determination module 28 of FIG. 1, in an embodiment.

In some embodiments, the velocity field and/or pressure field may be determined based only on the boundaries and the boundary conditions, without regard to time. For example, the velocity field and/or pressure field may be determined using a steady flow Navier-Stokes equation in which the velocity and pressure variables are functions of only spatial location (*i.e.*, time is not considered). This way, pressure and velocity can be accurately and efficiently determined in near real-time so as to enable point of care analysis by the clinician.

The method 40 may further include, at block 60, performing a virtual stress test based on the patient data and the computed fluid dynamics. Performing a virtual stress test may include calculating a set of pressure drops for one or more locations in the patient's vasculature across a range of activity levels-and, thus, a range of blood flow levels-of the patient. The set of pressure drops can be analyzed to determine if the patient has a clinically-significant pressure drop, indicating a stenosis, at an activity level that is relevant to the patient.

In some embodiments, block 60 may additionally or alternatively include calculating a reference pressure, an absolute pressure, and/or one or more hemodynamic index values across a range of activity levels. The absolute pressures and/or hemodynamic index values can be analyzed (*e.g.*, compared to a threshold) to determine if the patient has a clinically-significant pressure value or hemodynamic index value, indicating a stenosis, at a clinically-significant activity level.

The method 40 may further include, at block 54, recommending a further procedure for the patient based on the virtual stress test. For example, if the virtual stress test indicates a clinically-significant pressure drop at a location in the patient's vasculature for an activity level relevant to the patient, then a further diagnostic procedure may be recommended for the patient, such as an invasive angiography and pressure measurement at the site of the suspected stenosis. Additionally or alternatively, a corrective procedure may be recommended to address the stenosis, such as placement of a stent at the location of the suspected stenosis, for example.

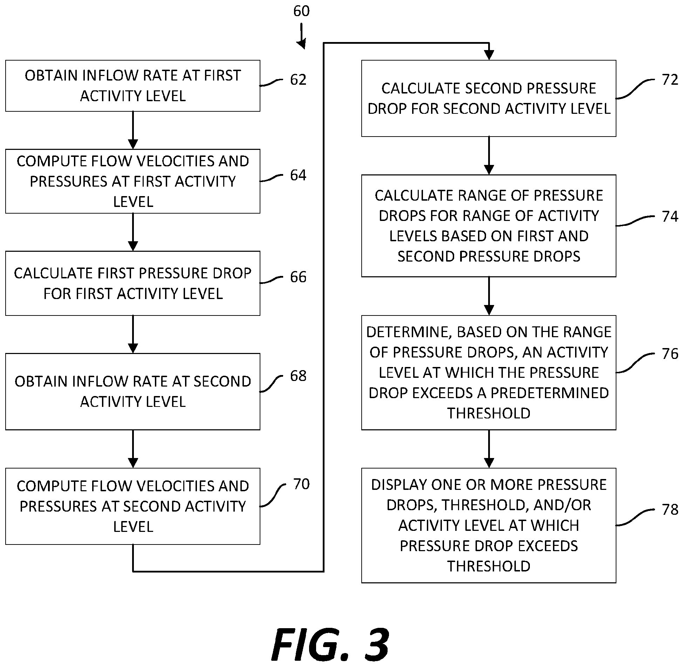

FIG. 3 is a flow chart illustrating an embodiment of a method 60 for performing a virtual stress test. The method 60, or one or more aspects of the method 60, may be performed by virtual stress test computing system 16 of FIG. 1, in embodiments. The method 60 may be considered an embodiment of blocks 48, 50, and 52 of the method 40.

The method 60 may include, at block 62, obtaining a first inflow rate at a first activity level of the patient. The first inflow rate may be an expected flow rate for the patient at a first physiological condition, for example, which may correlate with the first activity level. The first activity level may be any activity level within the relevant range of activity for the patient. For example, the first blood flow rate may be the patient's expected resting flow rate, corresponding to a low level of physical activity (that is, a state of rest). The flow rate at block 62 may be obtained via user manual entry of the flow rate (*e.g.*, with the user input device 14 of the system 10). In an embodiment, the flow rate at block 62 may be determined (*e.g.*, by a clinician or by an electronic system) based on the metabolic demands and condition of the patient (*e.g.*, according to equation (4) herein, determining a value of $\alpha$ appropriate for the first activity level). In an embodiment, the first inflow rate obtained at block 62 may be an arbitrary flow rate within a relevant range of flow rates for the patient (*i.e.*, a flow rate that the patient may be expected to experience during the patient's normal activities).

The method 60 may further include, at block 64, computing flow velocities and pressures in the region of interest at the first activity level of the patient. In some embodiments, the velocity field and/or pressure field may be determined based on defined boundaries and boundary conditions (which may, in turn, be based on the flow rate received at block 62) respective of the region of interest. For example, the velocity field and/or pressure field may be determined using steady flow Navier-Stokes equations in which the velocity and pressure variables are functions of only spatial location (i.e., time is not considered). The flow velocities and pressures may be determined, for example, by the flow field determination module 28 of the system 10 of FIG. 1.

The method 60 may further include, at block 66, calculating a first pressure drop (*e.g.*, at one or more user-designated points and/or other points) for the first activity level (*i.e.*, for the first inflow rate). The pressure drop may be a drop in pressure from the vessel inlet to a relevant point (*e.g.*, user-designated or otherwise). The pressure drop may be calculated based on a flow field calculated for the first activity level.

The method 60 may include, at block 68, obtaining a second inflow rate at a second activity level of the patient. The second inflow rate may be an expected flow rate for the patient at a second physiological condition, for example, which may correlate with the second activity level, and which second flow rate is different from the first inflow rate. The second activity level may be any activity level within the relevant range of activity for the patient. For example, the second blood flow rate may be the patient's expected hyperemic flow rate, corresponding to a high level of physical activity (that is, a state of maximum exertion). The flow rate at block 68 may be obtained via user manual entry of the flow rate (*e.g.*, with the user input device 14 of the system 10). In an embodiment, the flow rate at block 68 may be determined (*e.g.*, by a clinician or by an electronic system) based on the metabolic demands and condition of the patient (*e.g.*, according to equation (4) herein, determining a value of $\alpha$ appropriate for the second activity level). In an embodiment, the second inflow rate obtained at block 68 may be an arbitrary flow rate within a relevant range of flow rates for the patient (*i.e.*, a flow rate that the patient may be expected to experience during the patient's normal activities).

Blocks 62, 68 are described above with reference to an example in which the first flow rate represents a resting flow rate and the second flow rate represents a hyperemic flow rate. It should be noted, however, that the first and second flow rates obtained at blocks 62, 68 may be any two different flow rates within a range relevant to the patient's metabolic needs.

As discussed herein, a boundary condition flow rate may be specific to a portion or point in time in the cardiac cycle. For example, an inflow rate may be representative of an average flow rate over the entire cardiac cycle of the patient, or over the entire wave-free period of diastole of the patient, or over the entirety of diastole of the patient, or may be representative of the particular time point within the cardiac cycle, such as the midpoint of diastole. Accordingly, both the first and second inflow rates obtained at blocks 62, 68 may be representative of the same cardiac cycle portion or point, and the related boundary condition model value sets and subsequent calculated flow fields for the first and second inflow rates may similarly be representative of that same cardiac cycle portion or point.

The method 60 may further include, at block 70, computing flow velocities and pressures in the region of interest at the second activity level of the patient. The flow velocities and pressures may be computed substantially as described above with respect to block 64, but with the boundary conditions determined according to the second inflow rate.

The method 60 may further include, at block 72, calculating a second pressure drop at one or more points for the second activity level. The pressure drop may be calculated based on a flow field calculated for the second activity level at the one or more user-designated points.

The method 60 may further include, at block 74, calculating a range of pressure drops at the relevant (*e.g.*, user-designated) points for a range of activity levels based on the first and second pressure drops. The range of pressure drops may be calculated for a range of activity levels, from (or across) $Q_1$ to $Q_2$, where $Q_1$ is the first inflow rate and $Q_2$ is the second inflow rate. The pressure drop for a given flow rate may be calculated according to a quadratic equation, shown as equation (1) in this disclosure and repeated below:

$$\Delta P = aQ + bQ^2$$

where Q is the flow rate at the given activity level, and *a* and *b* are patient-specific constants. As described above, in order to compute *a* and *b,* three-dimensional Navier-Stokes equations can be solved for two values of Q (*e.g.*, the first and second inflow rates), as discussed herein with respect to equations (2) and (3). Additionally or alternatively, other computational methods can be used to solve for *a* and *b,* such as a Lattice-Boltzmann method or one-dimensional Navier-Stokes equations. Once coefficients *a* and *b* are known, ∆P may be calculated over a range of inflow rates (and, therefore, over a range of activity levels) without requiring CFD for each activity level.

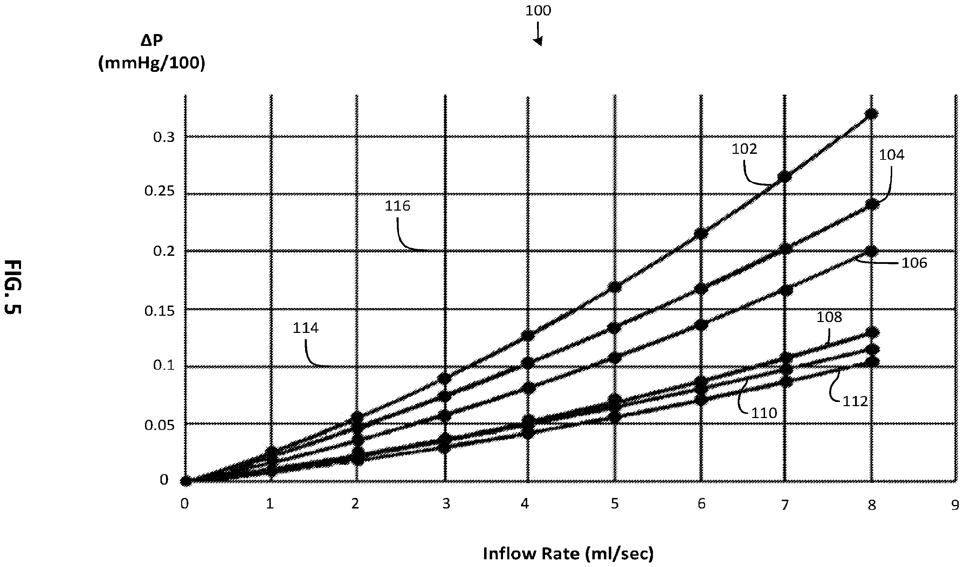

FIG. 5 is a plot 100 illustrating example ranges of pressure drops for example ranges of activity levels for a set of patients. The plot 100 of FIG. 5 includes pressure drop (in mmHg/100) on the vertical axis and flow rate (in ml/sec) on the horizontal axis, with six plot lines 102, 104, 106, 108, 110, 112 respective of six patients shown, and a horizontal threshold line 114 (shown at 20 mmHg) that may be indicative of a clinically significant pressure drop resulting from obstructive disease. In the plot 100, pressure drop plots respective of three patients-plot lines 102, 104, and 106-exceed the threshold at different flow rates, and thus at different levels of physical activity.

It should be noted that the pressure drop plot lines and threshold illustrated in FIG. 5 are only examples. In some embodiments, the illustrated threshold (20 mmHg), which corresponds approximately to an FFR of 08. Under hyperemic conditions, may be considered clinically relevant for a particular patient and therefore may be applied. In other embodiments, a value lower or higher than 20 mmHg may be considered clinically relevant, and thus may be applied as a threshold.

As noted above, the level of physical activity at which each patient's pressure drop range exceeds the threshold may or may not actually be relevant to that particular patient's lifestyle. For example, the patient represented by plot line 104 may live a very active lifestyle, and thus may regularly experience a high blood flow rate, and thus the pressure drop exceeding the threshold at a very high flow rate may be clinically significant for the patient. As a result, further diagnostic or interventional procedures may be recommended for the patient. In contrast, the patient represented by plot line 102 may live a sedentary lifestyle, and thus may rarely, if ever, experience a high blood flow rate, and thus the pressure drop exceeding the threshold at a somewhat high flow rate may not be clinically significant for the patient.

The method 60 may further include, at block 76, determining, based on the range of pressure drops, an activity level at which the pressure drop exceeds a threshold. The threshold may be set at a clinically-relevant pressure drop value, and may be selected manually based on clinical judgment, in embodiments. For example, the threshold may be selected according to a maximum MET value relevant to the patient's lifestyle, in some embodiments. The threshold may also be selected according to the cardiac cycle portion with respect to which the computations at blocks 64, 66, 70, 72 is made. Accordingly, in an embodiment, a threshold may be selected that is appropriate both for the patient's anticipated or estimated metabolic requirements, and also for the cardiac cycle portion for which analysis was performed in the method 60. The activity level at which the pressure drop of the user exceeds the threshold may then be compared to the patient's lifestyle and general level of activity to determine if further diagnostic or interventional procedures are warranted.

The method 60 may further include, at block 78, displaying the flow velocities and/or pressure drops at the first and/or second activity levels, and/or the flow threshold applied in block 76 or the activity level at which the patient's flow rate exceeds the threshold. The display may include, for example, one or more indicators of flow velocities and/or pressures at one or more locations in the patient's vasculature, overlaid on or presented adjacent to the geometric model of the patient anatomy.

As a result of the method 60, a flow field and/or pressure field may be calculated (and, in embodiments, displayed or otherwise output) for one or more locations in a patient's vasculature, for one or more cardiac cycle states of the patient. For example, in an embodiment, flow fields and pressure fields may be calculated and output for the beginning and end of diastole. Such flow fields may be used to determine hemodynamic information respective of the patient such as, for example, an IWFR value or other diastole-based hemodynamic value.

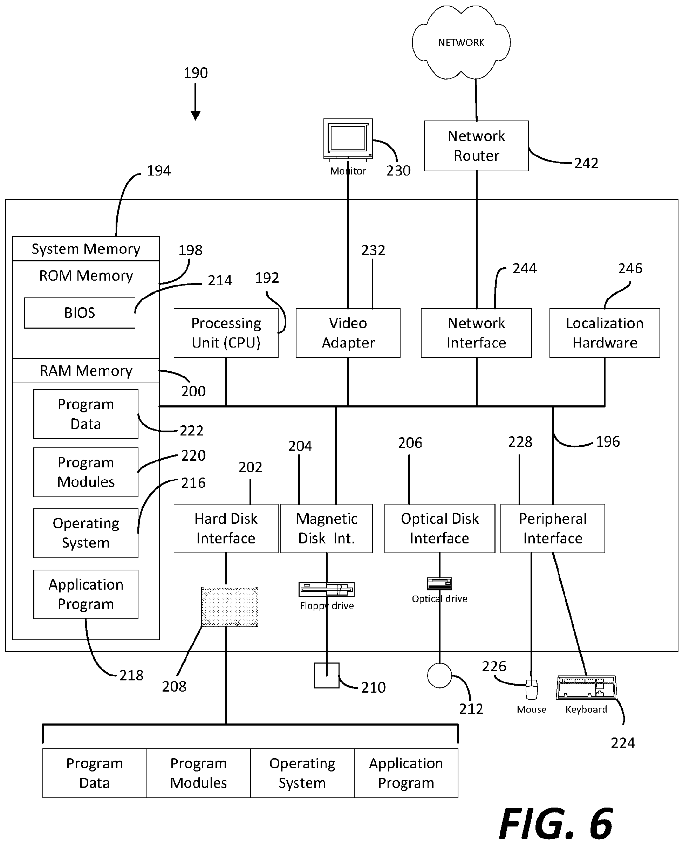

FIG. 6 is a diagrammatic view of an example embodiment of a user computing environment that includes a general purpose computing system environment 190, such as a desktop computer, laptop, smartphone, tablet, or any other such device having the ability to execute instructions, such as those stored within a non-transient, computer-readable medium. Furthermore, while described and illustrated in the context of a single computing system 190, those skilled in the art will also appreciate that the various tasks described hereinafter may be practiced in a distributed environment having multiple computing systems 190 linked via a local or wide-area network in which the executable instructions may be associated with and/or executed by one or more of multiple computing systems 190. The computing environment 190, or portions thereof, may comprise the system 10 of FIG. 1, in embodiments.

In its most basic configuration, computing system environment 190 typically includes at least one processing unit 192 and at least one memory 194, which may be linked via a bus 196. Depending on the exact configuration and type of computing system environment, memory 194 may be volatile (such as RAM 200), non-volatile (such as ROM 198, flash memory, etc.) or some combination of the two. Computing system environment 190 may have additional features and/or functionality. For example, computing system environment 190 may also include additional storage (removable and/or non-removable) including, but not limited to, magnetic or optical disks, tape drives and/or flash drives. Such additional memory devices may be made accessible to the computing system environment 190 by means of, for example, a hard disk drive interface 202, a magnetic disk drive interface 204, and/or an optical disk drive interface 206. As will be understood, these devices, which would be linked to the system bus 196, respectively, allow for reading from and writing to a hard disk 208, reading from or writing to a removable magnetic disk 210, and/or for reading from or writing to a removable optical disk 212, such as a CD/DVD ROM or other optical media. The drive interfaces and their associated computer-readable media allow for the nonvolatile storage of computer readable instructions, data structures, program modules and other data for the computing system environment 190. Those skilled in the art will further appreciate that other types of computer readable media that can store data may be used for this same purpose. Examples of such media devices include, but are not limited to, magnetic cassettes, flash memory cards, digital videodisks, Bernoulli cartridges, random access memories, nano-drives, memory sticks, other read/write and/or read-only memories and/or any other method or technology for storage of information such as computer readable instructions, data structures, program modules or other data. Any such computer storage media may be part of computing system environment 190.

A number of program modules may be stored in one or more of the memory/media devices. For example, a basic input/output system (BIOS) 214, containing the basic routines that help to transfer information between elements within the computing system environment 190, such as during start-up, may be stored in ROM 198. Similarly, RAM 200, hard drive 208, and/or peripheral memory devices may be used to store computer executable instructions comprising an operating system 216, one or more applications programs 218 (such as the modules 24, 26, 28, 30, 32 of FIG. 1), other program modules 220, and/or program data 222. Still further, computer-executable instructions may be downloaded to the computing environment 190 as needed, for example, via a network connection.

An end-user, e.g., a clinician, may enter commands and information into the computing system environment 190 through input devices such as a keyboard 224 and/or a pointing device 226. While not illustrated, other input devices may include a microphone, a joystick, a game pad, a scanner, etc. These and other input devices would typically be connected to the processing unit 192 by means of a peripheral interface 228 which, in turn, would be coupled to bus 196. Input devices may be directly or indirectly connected to processor 192 via interfaces such as, for example, a parallel port, game port, firewire, or a universal serial bus (USB). To view information from the computing system environment 190, a monitor 230 or other type of display device may also be connected to bus 196 via an interface, such as via video adapter 232. In addition to the monitor 230, the computing system environment 190 may also include other peripheral output devices, not shown, such as speakers and printers.

The computing system environment 190 may also utilize logical connections to one or more computing system environments. Communications between the computing system environment 190 and the remote computing system environment may be exchanged via a further processing device, such a network router 242, that is responsible for network routing. Communications with the network router 242 may be performed via a network interface component 244. Thus, within such a networked environment, e.g., the Internet, World Wide Web, LAN, or other like type of wired or wireless network, it will be appreciated that program modules depicted relative to the computing system environment 190, or portions thereof, may be stored in the memory storage device(s) of the computing system environment 190.

The computing system environment 190 may also include localization hardware 186 for determining a location of the computing system environment 190. In embodiments, the localization hardware 246 may include, for example only, a GPS antenna, an RFID chip or reader, a WiFi antenna, or other computing hardware that may be used to capture or transmit signals that may be used to determine the location of the computing system environment 190.

While this disclosure has described certain embodiments, it will be understood that the claims are not intended to be limited to these embodiments except as explicitly recited in the claims. On the contrary, the instant disclosure is intended to cover alternatives, and modifications, which may be included within the scope of the disclosure. Furthermore, in the detailed description of the present disclosure, numerous specific details are set forth in order to provide a thorough understanding of the disclosed embodiments. However, it will be obvious to one of ordinary skill in the art that systems and methods consistent with this disclosure may be practiced without these specific details. In other instances, well known methods, procedures, components, and circuits have not been described in detail as not to unnecessarily obscure various aspects of the present disclosure.

Some portions of the detailed descriptions of this disclosure have been presented in terms of procedures, logic blocks, processing, and other symbolic representations of operations on data bits within a computer or digital system memory. These descriptions and representations are the means used by those skilled in the data processing arts to most effectively convey the substance of their work to others skilled in the art. A procedure, logic block, process, etc., is herein, and generally, conceived to be a self-consistent sequence of steps or instructions leading to a desired result. The steps are those requiring physical manipulations of physical quantities. Usually, though not necessarily, these physical manipulations take the form of electrical or magnetic data capable of being stored, transferred, combined, compared, and otherwise manipulated in a computer system or similar electronic computing device. For reasons of convenience, and with reference to common usage, such data is referred to as bits, values, elements, symbols, characters, terms, numbers, or the like, with reference to various presently disclosed embodiments.

It should be borne in mind, however, that these terms are to be interpreted as referencing physical manipulations and quantities and are merely convenient labels that should be interpreted further in view of terms commonly used in the art. Unless specifically stated otherwise, as apparent from the discussion herein, it is understood that throughout discussions of the present embodiment, discussions utilizing terms such as "determining" or "outputting" or "transmitting" or "recording" or "locating" or "storing" or "displaying" or "receiving" or "recognizing" or "utilizing" or "generating" or "providing" or "accessing" or "checking" or "notifying" or "delivering" or the like, refer to the action and processes of a computer system, or similar electronic computing device, that manipulates and transforms data. The data is represented as physical (electronic) quantities within the computer system's registers and memories and is transformed into other data similarly represented as physical quantities within the computer system memories or registers, or other such information storage, transmission, or display devices as described herein or otherwise understood to one of ordinary skill in the art.

## Claims

1.  A computer-implemented method of performing a virtual stress test for a patient, the method comprising:

    receiving medical image data of a patient, the medical image data including an arterial segment;
    generating a geometric representation of the arterial segment from the medical image data;
    receiving metabolic requirement information of the patient;
    determining a threshold according to the metabolic requirement information;
    determining a first flow field for the arterial segment using the geometric representation, the first flow field corresponding to a first activity level of the patient;
    determining a first pressure drop in the arterial segment based on the first flow field;
    determining a second flow field for the arterial segment using the geometric representation, the second flow field corresponding to a second activity level of the patient that is different from the first activity level;
    determining a second pressure drop in the arterial segment based on the second flow field;
    calculating, based on the first pressure drop and the second pressure drop, a range of pressure drops for a range of activity levels;
    comparing the range of pressure drops to the threshold; and
    displaying a result of the comparison.

2.  The method of claim 1, wherein the first activity level is associated with a first flow rate and the second activity level is associated with a second flow rate that is different from the first flow rate, wherein calculating a range of pressure drops for the range of activity levels comprises calculating a range of pressure drops for a range of flow rates, andwherein the range of flow rates is between the first flow rate and the second flow rate.

3.  The method of claim 2, wherein calculating the range of pressure drops for the range of activity levels comprises calculating the range of pressure drops according to a quadratic relationship between pressure drop and flow rate.

4.  The method of claim 1, wherein displaying a result of the comparison comprises displaying a plot of the range of pressure drops and the threshold.

5. The method of any of claims 1-4, wherein the first activity level is a resting state and the second activity level is a hyperemic state.

6. The method of any of claims 1-4, further comprising determining an activity level at which the pressure drop exceeds the threshold.

7. The method of any of claims 1-4, wherein the first and second flow fields comprise first and second flow velocity fields, respectively.

8. The method of any of claims 1-4, wherein:

   determining the first flow field for the arterial segment using the geometric representation comprises:

   obtaining a first inflow rate respective of the arterial segment;
   calculating first outflow rates respective of the arterial segment according to the first inflow rate; and
   calculating the first flow field in the arterial segment according to the geometric representation, the first inflow rate, and the first outflow rates; and

   determining the second flow field for the arterial segment using the geometric representation comprises:

   obtaining a second inflow rate respective of the arterial segment;
   calculating second outflow rates respective of the arterial segment according to the second inflow rate; and
   calculating the second flow field in the arterial segment according to the geometric representation, the second inflow rate, and the second outflow rates.

9. A system comprising a processor and a non-transitory, computer-readable memory storing instructions that, when executed by the processor, cause the processor to:

   receive medical image data of a patient, the medical image data including an arterial segment;
   generate a geometric representation of the arterial segment from the medical image data
   receive metabolic requirement information of the patient;
   determine a threshold according to the metabolic requirement information;
   obtain a first inflow rate respective of the inflow boundary;
   determining a first flow field for the arterial segment using the geometric representation, the first flow field corresponding to a first activity level of the patient;
   determining a first pressure drop in the arterial segment based on the first flow field;
   determining a second flow field for the arterial segment using the geometric representation, the second flow field corresponding to a second activity level of the patient that is different from the first activity level;
   determining a second pressure drop in the arterial segment based on the second flow field;
   calculating, based on the first pressure drop and the second pressure drop, a range of pressure drops for a range of inflow rates;
   comparing the range of pressure drops to the threshold; and
   displaying a result of the comparison.

10. The system of claim 9, wherein calculating a range of pressure drops for the range of flow rates comprises calculating a range of pressure drops for a range of activity levels, and wherein the range of flow rates is between the first flow rate and the second flow rate.

11. The system of claim 9 or claim 10, wherein calculating the range of pressure drops comprises calculating the range of pressure drops according to a quadratic relationship between pressure drop and inflow rate; or
    wherein displaying a result of the comparison comprises displaying a plot of the range of pressure drops and the threshold.

12. The system of claim 9 or claim 10, further comprising determining an activity level at which the pressure drop exceeds the threshold.

13. The system of claim 9 or claim 10, wherein the first flow field is respective of a resting state of the patient and the second flow field is respective of a hyperemic state of the patient.

14. The system of claim 9 or claim 10, wherein the first and second flow fields comprise first and second flow velocity fields, respectively.

15. The system of claim 9 or claim 10, wherein the first and second flow fields are calculated according to three-dimensional, time-independent equations.

**Patentansprüche**

1. Computerimplementiertes Verfahren zur Durchführung eines virtuellen Belastungstests für einen Patienten, wobei das Verfahren umfasst:

    Empfangen medizinischer Bilddaten eines Patienten, wobei die medizinischen Bilddaten ein Arteriensegment einschließen;
    Erzeugen einer geometrischen Darstellung des Arteriensegments aus den medizinischen Bilddaten;
    Empfangen von Informationen über den Stoffwechselbedarf des Patienten;
    Bestimmen einer Schwelle gemäß den Informationen über den Stoffwechselbedarf;
    Bestimmen eines ersten Flussfeldes für das Arteriensegment unter Verwendung der geometrischen Darstellung, wobei das erste Flussfeld einem ersten Aktivitätsniveau des Patienten entspricht;
    Bestimmen eines ersten Druckabfalls im Arteriensegment basierend auf dem ersten Flussfeld;
    Bestimmen eines zweiten Flussfeldes für das Arteriensegment unter Verwendung der geometrischen Darstellung, wobei das zweite Flussfeld einem zweiten Aktivitätsniveau des Patienten entspricht, das sich vom ersten Aktivitätsniveau unterscheidet;
    Bestimmen eines zweiten Druckabfalls im Arteriensegment basierend auf dem zweiten Flussfeld;
    Berechnen, auf Grundlage des ersten Druckabfalls und des zweiten Druckabfalls, eines Bereichs von Druckabfällen für einen Bereich von Aktivitätsniveaus;
    Vergleichen des Bereichs von Druckabfällen mit der Schwelle; und
    Anzeigen eines Ergebnisses des Vergleichs.

2. Verfahren nach Anspruch 1, wobei das erste Aktivitätsniveau einer ersten Durchflussrate zugeordnet ist und das zweite Aktivitätsniveau einer zweiten Durchflussrate zugeordnet ist, die sich von der ersten Durchflussrate unterscheidet, wobei das Berechnen eines Bereichs von Druckabfällen für den Bereich von Aktivitätsniveaus das Berechnen eines Bereichs von Druckabfällen für einen Bereich von Durchflussraten umfasst, und wobei der Bereich von Durchflussraten zwischen der ersten Durchflussrate und der zweiten Durchflussrate liegt.

3. Verfahren nach Anspruch 2, wobei das Berechnen des Bereichs von Druckabfällen für den Bereich von Aktivitätsniveaus das Berechnen des Bereichs von Druckabfällen gemäß einer quadratischen Beziehung zwischen Druckabfall und Durchflussrate umfasst.

4. Verfahren nach Anspruch 1, wobei das Anzeigen eines Ergebnisses des Vergleichs das Anzeigen einer Grafik des Bereichs von Druckabfällen und der Schwelle umfasst.

5. Verfahren nach einem der Ansprüche 1 bis 4, wobei das erste Aktivitätsniveau ein Ruhezustand ist und das zweite Aktivitätsniveau ein hyperämischer Zustand ist.

6. Verfahren nach einem der Ansprüche 1 bis 4, weiter umfassend das Bestimmen eines Aktivitätsniveaus, bei dem der Druckabfall die Schwelle überschreitet.

7. Verfahren nach einem der Ansprüche 1 bis 4, wobei das erste und das zweite Flussfeld ein erstes bzw. ein zweites Flussgeschwindigkeitsfeld umfassen.

8. Verfahren nach einem der Ansprüche 1 bis 4, wobei:

    das Bestimmen des ersten Flussfeldes für das Arteriensegment unter Verwendung der geometrischen Darstellung umfasst:

        Erhalten einer ersten Zuflussrate zugehörig zum Arteriensegment;
        Berechnen erster Abflussraten zugehörig zum Arteriensegment gemäß der ersten Zuflussrate; und

Berechnen des ersten Flussfeldes in dem Arteriensegment gemäß der geometrischen Darstellung, der ersten Zuflussrate und der ersten Abflussraten; und

das Bestimmen des zweiten Flussfeldes für das Arteriensegment unter Verwendung der geometrischen Darstellung umfasst:

Erhalten einer zweiten Zuflussrate zugehörig zum Arteriensegment;
Berechnen zweiter Abflussraten zugehörig zum Arteriensegment gemäß der zweiten Zuflussrate; und
Berechnen des zweiten Flussfeldes in dem Arteriensegment gemäß der geometrischen Darstellung, der zweiten Zuflussrate und der zweiten Abflussraten.

9. System, das einen Prozessor und einen nicht-transitorischen, computerlesbaren Speicher umfasst, der Anweisungen speichert, die, wenn sie von dem Prozessor ausgeführt werden, den Prozessor veranlassen zum:

Empfangen medizinischer Bilddaten eines Patienten, wobei die medizinischen Bilddaten ein Arteriensegment einschließen;
Erzeugen einer geometrischen Darstellung des Arteriensegments aus den medizinischen Bilddaten, Empfangen von Informationen über den Stoffwechselbedarf des Patienten;
Bestimmen einer Schwelle gemäß den Informationen über den Stoffwechselbedarf;
Erhalten einer ersten Zuflussrate zugehörig zur Zuflussgrenze;
Bestimmen eines ersten Flussfeldes für das Arteriensegment unter Verwendung der geometrischen Darstellung, wobei das erste Flussfeld einem ersten Aktivitätsniveau des Patienten entspricht;
Bestimmen eines ersten Druckabfalls im Arteriensegment basierend auf dem ersten Flussfeld;
Bestimmen eines zweiten Flussfeldes für das Arteriensegment unter Verwendung der geometrischen Darstellung, wobei das zweite Flussfeld einem zweiten Aktivitätsniveau des Patienten entspricht, das sich vom ersten Aktivitätsniveau unterscheidet;
Bestimmen eines zweiten Druckabfalls im Arteriensegment basierend auf dem zweiten Flussfeld;
Berechnen, auf Grundlage des ersten Druckabfalls und des zweiten Druckabfalls, eines Bereichs von Druckabfällen für einen Bereich von Zuflussraten;
Vergleichen des Bereichs von Druckabfällen mit der Schwelle; und
Anzeigen eines Ergebnisses des Vergleichs.

10. System nach Anspruch 9, wobei das Berechnen eines Bereichs von Druckabfällen für den Bereich von Durchflussraten das Berechnen eines Bereichs von Druckabfällen für einen Bereich von Aktivitätsniveaus umfasst, und wobei der Bereich von Durchflussraten zwischen der ersten Durchflussrate und der zweiten Durchflussrate liegt.

11. System nach Anspruch 9 oder Anspruch 10, wobei das Berechnen des Bereichs von Druckabfällen das Berechnen des Bereichs von Druckabfällen gemäß einer quadratischen Beziehung zwischen Druckabfall und Zuflussrate umfasst; oder
wobei das Anzeigen eines Ergebnisses des Vergleichs das Anzeigen einer Grafik des Bereichs von Druckabfällen und der Schwelle umfasst.

12. System nach Anspruch 9 oder Anspruch 10, weiter umfassend das Bestimmen eines Aktivitätsniveaus, bei dem der Druckabfall die Schwelle überschreitet.

13. System nach Anspruch 9 oder Anspruch 10, wobei das erste Flussfeld einem Ruhezustand des Patienten zugehört und das zweite Flussfeld einem hyperämischer Zustand des Patienten zugehört.

14. System nach Anspruch 9 oder Anspruch 10, wobei das erste und das zweite Flussfeld ein erstes bzw. ein zweites Flussgeschwindigkeitsfeld umfassen.

15. System nach Anspruch 9 oder Anspruch 10, wobei das erste und das zweite Flussfeld gemäß dreidimensionalen, zeitunabhängigen Gleichungen berechnet werden.

**Revendications**

1. Procédé mis en oeuvre par ordinateur pour effectuer un test de contrainte virtuel pour un patient, le procédé comprenant :

la réception de données d'image médicale d'un patient, les données d'image médicale incluant un segment artériel ;

la génération d'une représentation géométrique du segment artériel à partir des données d'image médicale ;

la réception d'informations sur les besoins métaboliques du patient ;

la détermination d'un seuil en fonction des informations sur les besoins métaboliques ;

la détermination d'un premier champ de flux pour le segment artériel à l'aide de la représentation géométrique, le premier champ de flux correspondant à un premier niveau d'activité du patient ;

la détermination d'une première chute de pression dans le segment artériel sur la base du premier champ de flux ;

la détermination d'un second champ de flux pour le segment artériel à l'aide de la représentation géométrique, le second champ de flux correspondant à un second niveau d'activité du patient qui est différent du premier niveau d'activité ;

la détermination d'une seconde chute de pression dans le segment artériel sur la base du second champ de flux ;

le calcul, sur la base de la première chute de pression et de la seconde chute de pression, d'une plage de chutes de pression pour une plage de niveaux d'activité ;

la comparaison de la plage de chutes de pression au seuil ; et

l'affichage d'un résultat de la comparaison.

2. Procédé selon la revendication 1, dans lequel le premier niveau d'activité est associé à un premier débit et le second niveau d'activité est associé à un second débit qui est différent du premier débit, dans lequel le calcul d'une plage de chutes de pression pour la plage de niveaux d'activité comprend le calcul d'une plage de chutes de pression pour une plage de débits, et dans lequel la plage de débits se situe entre le premier débit et le second débit.

3. Procédé selon la revendication 2, dans lequel le calcul de la plage de chutes de pression pour la plage de niveaux d'activité comprend le calcul de la plage de chutes de pression selon une relation quadratique entre chute de pression et débit.

4. Procédé selon la revendication 1, dans lequel l'affichage d'un résultat de la comparaison comprend l'affichage d'un tracé de la plage de chutes de pression et du seuil.

5. Procédé selon l'une quelconque des revendications 1-4, dans lequel le premier niveau d'activité est un état de repos et le second niveau d'activité est un état hyperémique.

6. Procédé selon l'une quelconque des revendications 1-4, comprenant en outre la détermination d'un niveau d'activité auquel la chute de pression dépasse le seuil.

7. Procédé selon l'une quelconque des revendications 1-4, dans lequel les premier et second champs de flux comprennent respectivement des premier et second champs de vitesse de flux.

8. Procédé selon l'une quelconque des revendications 1-4, dans lequel :

la détermination du premier champ de flux pour le segment artériel à l'aide de la représentation géométrique comprend :

l'obtention d'un premier débit d'entrée respectif du segment artériel ;

le calcul de premiers débits de sortie respectifs du segment artériel en fonction du premier débit d'entrée ; et

le calcul du premier champ de flux dans le segment artériel en fonction de la représentation géométrique, du premier débit d'entrée et des premiers débits de sortie ; et

la détermination du second champ de flux pour le segment artériel à l'aide de la représentation géométrique comprend :

l'obtention d'un second débit d'entrée respectif du segment artériel ;

le calcul de seconds débits de sortie respectifs du segment artériel en fonction du second débit d'entrée ; et

le calcul du second champ de flux dans le segment artériel en fonction de la représentation géométrique, du second débit d'entrée et des seconds débits de sortie.

9. Système comprenant un processeur et une mémoire non transitoire lisible par ordinateur stockant des instructions qui, lorsqu'elles sont exécutées par le processeur, amènent le processeur à :

recevoir des données d'image médicale d'un patient, les données d'image médicale incluant un segment artériel ;

générer une représentation géométrique du segment artériel à partir des données d'image médicale et recevoir des informations sur les besoins métaboliques du patient ;

déterminer un seuil en fonction des informations sur les besoins métaboliques ;

obtenir un premier débit d'entrée respectif de la limite de flux d'entrée ;

déterminer un premier champ de flux pour le segment artériel à l'aide de la représentation géométrique, le premier champ de flux correspondant à un premier niveau d'activité du patient ;

déterminer une première chute de pression dans le segment artériel sur la base du premier champ de flux ;

déterminer un second champ de flux pour le segment artériel à l'aide de la représentation géométrique, le second champ de flux correspondant à un second niveau d'activité du patient qui est différent du premier niveau d'activité ;

déterminer une seconde chute de pression dans le segment artériel sur la base du second champ de flux ;

calculer, sur la base de la première chute de pression et de la seconde chute de pression, une plage de chutes de pression pour une plage de débits d'entrée ;

comparer la plage de chutes de pression au seuil ; et

afficher un résultat de la comparaison.

10. Système selon la revendication 9, dans lequel le calcul d'une plage de chutes de pression pour la plage de débits comprend le calcul d'une plage de chutes de pression pour une plage de niveaux d'activité, et dans lequel la plage de débits se situe entre le premier débit et le second débit.

11. Système selon la revendication 9 ou la revendication 10, dans lequel le calcul de la plage de chutes de pression comprend le calcul de la plage de chutes de pression selon une relation quadratique entre chute de pression et débit d'entrée ; ou

dans lequel l'affichage d'un résultat de la comparaison comprend l'affichage d'un tracé de la plage de chutes de pression et du seuil.

12. Système selon la revendication 9 ou la revendication 10, comprenant en outre la détermination d'un niveau d'activité auquel la chute de pression dépasse le seuil.

13. Système selon la revendication 9 ou la revendication 10, dans lequel le premier champ de flux est respectif d'un état de repos du patient et le second champ de flux est respectif d'un état hyperémique du patient.

14. Système selon la revendication 9 ou la revendication 10, dans lequel les premier et second champs de flux comprennent respectivement des premier et second champs de vitesse de flux.

15. Système selon la revendication 9 ou la revendication 10, dans lequel les premier et second champs de flux sont calculés selon des équations tridimensionnelles indépendantes du temps.

10

PATIENT
IMAGE
SOURCE    12

USER INPUT
DEVICE    14

VIRTUAL STRESS TEST COMPUTING SYSTEM

PROCESSOR    20

MEMORY

GEOMETRY
DETERMINATION    24

BOUNDARY
CONDITION
DETERMINATION    26

FLOW FIELD
DETERMINATION    28

PRESSURE
DETERMINATION    22    30

VIRTUAL STRESS
TEST    32

16

DISPLAY    18

**FIG. 1**

40

RECEIVE PATIENT DATA — 42

RECEIVE IMAGES OF PATIENT ANATOMY — 44

CREATE ANATOMICAL MODEL BASED ON IMAGES — 46

DETERMINE ONE OR MORE BOUNDARY CONDITIONS MODEL VALUE SETS

OBTAIN INFLOW RATE — 48a

— 48

CALCULATE OUTLET FLOW RATES BASED ON INFLOW RATE AND FLOW SPLIT MODEL — 48b

COMPUTE FLUID DYNAMICS BASED ON ANATOMICAL MODEL AND BOUNDARY CONDITIONS — 50

PERFORM VIRTUAL STRESS TEST BASED ON PATIENT DATA AND COMPUTED FLUID DYNAMICS — 52

RECOMMEND FURTHER PROCEDURE BASED ON VIRTUAL STRESS TEST — 54

*FIG. 2*

60

| OBTAIN INFLOW RATE AT FIRST ACTIVITY LEVEL | 62 |

↓

| COMPUTE FLOW VELOCITIES AND PRESSURES AT FIRST ACTIVITY LEVEL | 64 |

↓

| CALCULATE FIRST PRESSURE DROP FOR FIRST ACTIVITY LEVEL | 66 |

↓

| OBTAIN INFLOW RATE AT SECOND ACTIVITY LEVEL | 68 |

↓

| COMPUTE FLOW VELOCITIES AND PRESSURES AT SECOND ACTIVITY LEVEL | 70 |

| CALCULATE SECOND PRESSURE DROP FOR SECOND ACTIVITY LEVEL | 72 |

↓

| CALCULATE RANGE OF PRESSURE DROPS FOR RANGE OF ACTIVITY LEVELS BASED ON FIRST AND SECOND PRESSURE DROPS | 74 |

↓

| DETERMINE, BASED ON THE RANGE OF PRESSURE DROPS, AN ACTIVITY LEVEL AT WHICH THE PRESSURE DROP EXCEEDS A PREDETERMINED THRESHOLD | 76 |

↓

| DISPLAY ONE OR MORE PRESSURE DROPS, THRESHOLD, AND/OR ACTIVITY LEVEL AT WHICH PRESSURE DROP EXCEEDS THRESHOLD | 78 |

*FIG. 3*

58

**FIG. 4**

FIG. 5

190

NETWORK

194

Network
Router

242

Monitor 230

System Memory 198

ROM Memory 214 192

244 246

BIOS

Processing
Unit (CPU)

Video
Adapter

Network
Interface

Localization
Hardware

RAM Memory 200

Program
Data 222

232

Program
Modules 220

216

204 206 228 196

Operating
System

202

Hard Disk
Interface

Magnetic
Disk  Int.

Optical Disk
Interface

Peripheral
Interface

Application
Program

Floppy drive

Optical drive

218 208

210

226

212

Mouse Keyboard

224

| Program Data | Program Modules | Operating System | Application Program |
|---|---|---|---|

*FIG. 6*

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 62788911

- US 2018368916 A

**Non-patent literature cited in the description**

- **SIDNEY et al.** Metabolic Equivalents (METS) in Exercise Testing, Exercise Prescription, and Evaluation of Functional Capacity. *13 Clin. Cardiol,* 1990, 555-565

- **LEAR et al.** Exercise Stress Testing: An Overview of Current Guidelines. *Sports Med,* May 1999, vol. 27 (5), 285-312